(19) Europäisches Patentamt
European Patent Office
Office européen des brevets

(11) Publication number : **0 380 359 B1**

(12) **EUROPEAN PATENT SPECIFICATION**

(45) Date of publication of patent specification : **29.12.93 Bulletin 93/52**

(51) Int. Cl.$^5$ : **A01N 57/34,** A61K 31/66

(21) Application number : **90300845.6**

(22) Date of filing : **26.01.90**

(54) **Biocidal compositions and treatments.**

(30) Priority : **27.01.89 GB 8901881**

(43) Date of publication of application : **01.08.90 Bulletin 90/31**

(45) Publication of the grant of the patent : **29.12.93 Bulletin 93/52**

(84) Designated Contracting States : **AT BE CH DE DK ES FR GB GR IT LI LU NL SE**

(56) References cited :
**GB-A- 2 178 960**
**GB-A- 2 182 563**
**GB-A- 2 201 592**

(73) Proprietor : **Albright & Wilson Limited**
**210-222 Hagley Road West**
**Oldbury Warley West Midlands B68 0NN (GB)**

(72) Inventor : **Hoye, Peter Albert Theodore**
**Tresco, Orchard Grove, Kinver**
**Stourbridge, SWest Midlands DY1 6LL (GB)**

(74) Representative : **Savidge, Roger Gordon Madgwick et al**
**Albright & Wilson Limited Patents Department**
**210-222 Hagley Road West Oldbury**
**Warley, West Midlands B68 ONN (GB)**

EP 0 380 359 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to biocidal compositions comprising lower alkyl phosphonium salts and biocidal treatments therewith.

The use of a group of lower alkyl hydroxymethyl phosphonium salts in general and of tetrakis hydroxymethyl phosphonium (herein referred to as "THP") salts in particular for water treatment, plant protection, and for pharmaceutical and veterinary treatments is known from GB 2145708, GB-A-2178960, GB-A-2182563, GB-A-2201592 and GB-A-2205310. Hitherto it has been believed that THP salts were very much more active as biocides than any other members of the group.

The use of long chain alkyl phosphonium salts for water treatment is known from EP066544. These have been shown to be substantially less effective as biocides than THP salts. Other patents which disclose biocidal activity of organophosphonium compounds include US-A-4015 037, FR-A-2341 689, JP-A-75 136 500, EP-A-105843, GB-A-2136 433, EP-A-0105843 and US-A-3013085. In each case the organophosphonium ions specifically described have been shown to be inactive per se or less active per se than THP and/or are dependent for their activity on the presence of a biocidal anion.

We have now found that certain lower alkyl phosphonium salts selected from the general group disclosed in GB 2145708 are especially effective as biocides, being under certain conditions more active than THP salts themselves.

Our invention provides the biocidal use of phosphonium salts of the formula $[RP(CH_2OH)_3]^{+}\frac{1}{V}X^{(v-)}$ wherein R is a methyl, ethyl or allyl group, and X is an anion having valency v such that the salt is water soluble. The salts for use according to our invention are thus methyl tris (hydroxymethyl) phosphonium, ethyl tris (hydroxymethyl) phosphonium and allyl tris (hydroxymethyl) phosphonium.

The invention also provides as novel biocidal compounds the bis phosphonium products of the formula

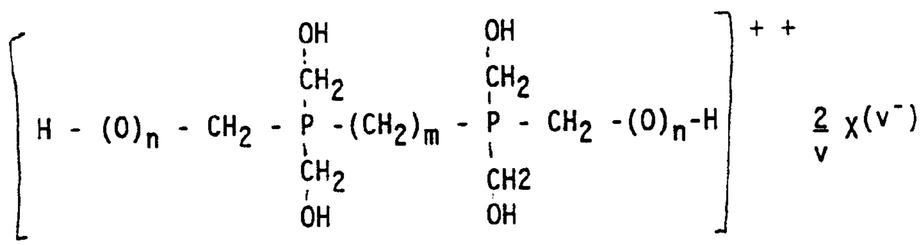

where n is 0 or 1, m is the valency of the anion X.

The preferred novel compounds of the aforesaid formula are water soluble salts of

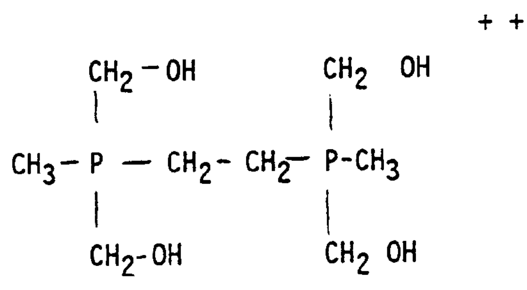

or of

$$(HOCH_2)_3\,P\,CH_2\,CH_2\,P\,(CH_2\,OH)_3^{++}$$

cations, especially the halide salts such as chlorides and bromides.

The anion X in each of the above formulae may be any convenient anion which provides a salt which is soluble at least to a concentration of 0.5gm per litre at 25°C such as chloride, sulphate or phosphate or less preferably sulphite, phosphite, bromide, nitrate, borate, acetate, formate, lactate, methosulphate, citrate or carbonate. The salts of the aforesaid anions are novel, constituting a further aspect of the invention.

The invention also covers the biocidal use of condensates of the aforesaid phosphonium salts with ammonia or an amine or amide such as urea, melamine, thiourea, guanidine or dicyandiamide.

The biocides of our invention are useful for treating aerobic or anaerobic water systems contaminated or liable to be contaminated with microorganisms. For example they are effective against Pseudomonas aeruginosa, Legionella pneumophilla and planktonic algae in boiler water, cooling water, industrial process water, geothermal water, central heating and air conditioning systems, for killing algae in swimming pools, fish ponds, aquaria, fish farms, lakes, streams, canals and reservoirs and for treating cooling water in power stations and for marine engines.

The biocides are also useful in killing sulphate reducing bacteria such as Desulphovibrio in oil field produced water, injection water, drilling fluids or water for hydrostatic testing. They are also useful as preservatives in aqueous based formulations such as bitumen and tar emulsions, paper sizes, adhesives, paints, pharmaceutical and cosmetic formulations.

The biocides are useful in disinfectants including farmyard, domestic and surgical disinfectants. They may be used in the fumigation of grain silos, crops and crop storage areas.

The biocides are useful for killing bryophites, including mosses and liverworts, lichens, and sessile algae in lawns and gardens and on paths, drives, roadways, walls and other structures and on railways, airports and industrial estates.

The biocides are useful for protecting plants against fungi, bacteria, viruses and other microbial plant pathogens, by application to the plants and or to the soil in which they are growing or to be grown, or for use in a seed dressing.

The biocides are useful for the pharmaceutical treatment of humans and the veterinary treatment of animals, e.g. in topical applications for prevention or cure of athletes foot, ringworm, thrush or other fungal or yeast infections, boils, anthrax and other bacterial skin infections, and for oral or parenteral treatment of protozoal, systemic fungal, bacterial, rickettsial and viral infections.

The selective activity of the biocides is concentration dependent. Generally at concentrations of between 10 and 2,000 ppm preferably 20 to 1,500 eg 30 to 1,000 especially 50 to 500ppm the biocides show selective activity against lower organisms such as bacteria, algae, mosses and fungi, but exhibit very low toxicity towards higher plants, fish and mammals. At higher concentrations, e.g greater than 0.2% up to saturation, preferably 0.5 to 75% e.g. 1 to 60% and at dosages of greater than about 2 kg per hectare e.g. 2.5 to 5kg per hectare the biocides are, however, effective total herbicides.

Mixed alkyl hydroxyalkyl THP salts may be prepared by adding an aqueous base to a tetrakis (hydroxymethyl) phosphonium salt, e.g. sodium hydroxide in a proportion of from 0.5 to 0.75 equivalents, to form tris (hydroxymethyl) phosphine and reacting the latter with alkyl halide such as methylchloride, preferably at an elevated temperature of e.g. 40-60°C. Alternatively, improved yields may be obtained by reacting the alkylhalide with tris (acetoxymethyl) phosphine prepared by the method of Mironova et al, Zhur. Obshch. Khim. 37, No 12, pages 2747 - 2752. The reaction may be carried out by heating at temperatures up to 140°, eg 120°C in suitable solvent such as toluene, for 2 to 20 hours eg 10 to 15 hours. The acetate product is hydrolysed with an acid catalyst, preferably acetic acid, for from 1 to 8 hours, e.g. 3 to 5 hours.

The novel bis phosphonium salts in which n is 0 may be made by reaction of an ester, eg a carboxylate ester, of methyl bis (hydroxy methyl) phosphine such as methyl bis (acetoxy methyl) phosphine, with an alpha omega alkylene dihalide eg a chloride or iodide or especially a bromide usually with heating eg at 50 - 150°C for 5 - 30 hours, to produce the esterified derivatives of said biocidal compounds, which are then hydrolysed eg in dilute acid usually at 0 - 100°C for 1 - 24 hours to produce a solution of the salts, from which solid salts may be obtained by crystallisation, if required.

The novel bis phosphonium salts in which n is 1 may be made by preparation of an alpha omega alkylene bis phosphonous dihalide, followed by reduction of the bis phosphonous dihalide with lithium aluminium hydride eg in an ethereal solvent and especially at -10°C to +20°C for 0.1 - 24 hours to form the alpha omega alkylene bis phosphine, which is then reacted with formaldehyde in the presence of an acid eg hydrochloric acid usually at 20 - 100°C for 0.1 - 2 hours to form the alpha omega alkylene bis (tris hydroxy methyl phosphonium) salt, from which solid salts may be obtained by crystallisation, if required.

The invention provides compositions containing the aforesaid biocides. In particular for use in water treatment and in agriculture we have found that the biocides are synergistic with surfactants.

The surfactant may for example consist substantially of an at least sparingly water-soluble salt of sulphonic or mono esterified sulphuric acids, e.g. an alkylbenzene sulphonate, alkyl sulphate, alkyl ether sulphate, olefin sulphonate, alkane sulphonate, alkylphenol sulphate, alkylphenol ether sulphate, alkylethanolamide sulphate, alkylethanolamide ether sulphate, or alpha sulpho fatty acid or its esters each having at least one alkyl or alkenyl group with from 8 to 22, more usually 10 to 20, aliphatic carbon atoms.

The expression "ether" hereinbefore refers to compounds containing one or more glyceryl groups and/or an oxyalkylene or polyoxyalkylene group especially a group containing from 1 to 20 oxyethylene and/or oxypropylene groups. One or more oxybutylene groups may additionally or alternatively be present. For example, the sulphonated or sulphated surfactant may be sodium dodecyl benzene sulphonate, potassium hexadecyl benzene sulphonate, sodium dodecyl dimethyl benzene sulphonate, sodium lauryl sulphate, sodium tallow sulphate, potassium oleyl sulphate, ammonium lauryl monoethoxy sulphate, or monoethanolamine cetyl 10 mole ethoxylate sulphate.

Other anionic surfactants useful according to the present invention include alkyl sulphosuccinates, such as sodium di-2-ethylhexylsulphosuccinate and sodium dihexylsulphosuccinate, alkyl ether sulphosuccinates, alkyl sulphosuccinamates, alkyl ether sulphosuccinamates, acyl sarcosinates, acyl taurides, isethionates, soaps such as stearates, palmitates, resinates, oleates, linoleates, and alkyl ether carboxylates. Anionic phosphate esters and alkyl phosphonates, alkyl amino and imino methylene phosphonates may also be used. In each case the anionic surfactant typically contains at least one aliphatic hydrocarbon chain having from 8 to 22, preferably 10 to 20 carbon atoms, and, in the case of ethers, one or more glyceryl and/or from 1 to 20 oxyethylene and/or oxypropylene and/or oxybutylene groups.

Preferred anionic surfactants are sodium salts. Other salts of commercial interest include those of potassium, lithium, calcium, magnesium, ammonium, monoethanolamine, diethanolamine, triethanolamine, alkyl amines containing up to seven aliphatic carbon atoms, and quaternary ammonium or phosphonium such as alkyl and/or hydroxyalkyl phosphonium.

The surfactant may optionally contain or consist of nonionic surfactants. The nonionic surfactant may be, e.g. a $C_{10-22}$ alkanolamide of a mono or di- lower alkanolamine, such as coconut monoethanolamide. Other nonionic surfactants which may optionally be present, include tertiary acetylenic glycols, polyethoxylated alcohols, polyethoxylated mercaptans, polyethoxylated carboxylic acids, polyethoxylated amines, polyethoxylated alkylolamides, polyethoxylated alkylphenols, polyethoxylated glyceryl esters, polyethoxylated sorbitan esters, polyethoxylated phosphate esters, and the propoxylated or ethoxylated and propoxylated analogues of all the aforesaid ethoxylated nonionics, all having a $C_{8-22}$ alkyl or alkenyl group and up to 20 ethyleneoxy and/or propyleneoxy groups.

Also included are polyoxypropylene/polyoxethylene copolymers, polyoxybutylene/polyoxyethylene copolymers and polyoxybutylene/polyoxypropylene copolymers. The polyoxyethylene polyoxypropylene and polyoxybutylene compounds may optionally be end-capped with, e.g. benzyl groups to reduce their foaming tendency.

Compositions of our invention may contain amphoteric surfactant.

The amphoteric surfactant may for example be a betaine, e.g. a betaine of the formula:- $R_3N^+CH_2COO^-$, wherein each R is an alkyl, cycloalkyl, alkenyl or alkaryl group and preferably at least one, and most preferably not more than one R, has an average of from 8 to 20, e.g. 10 to 18 aliphatic carbon atoms and each other R has an average of from 1 to 4 carbon atoms. Particularly preferred are the quaternary imidazoline betaines of the formula:

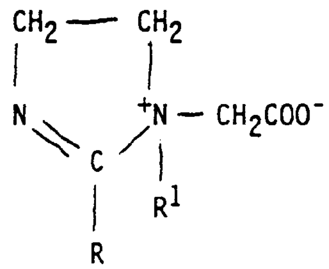

wherein R and $R^1$ are alkyl, alkenyl, cycloalkyl, alkaryl or alkanol groups having an average of from 1 to 20 aliphatic carbon atoms and R preferably has an average of from 8 to 20, e.g. 10 to 18 aliphatic carbon atoms and $R^1$ preferably has 1 to 4 carbon atoms. Other amphoteric surfactants for use according to our invention include alkyl amine ether sulphates, sulphobetaines and other quaternary amine or quaternised imidazoline sulphonic acids and their salts, and other quaternary amine or quaternised imidazoline carboxylic acids and their salts and Zwitterionic surfactants, e.g. N-alkyl taurines, carboxylated amido amines such as $RCONH(CH_2)_2N^+(CH_2CH_2CH_3)_2CH_2CO_2$, and amino acids having, in each case, hydrocarbon groups capable of conferring surfactant properties (e.g. alkyl, cycloalkyl, alkenyl or alkaryl groups having from 8 to 20 aliphatic carbon atoms).

Typical examples include 2 tallow alkyl, 1-tallow amido alkyl, 1-carboxymethyl imidazoline and 2 coconut alkyl N-carboxymethyl 2-(hydroxyalkyl) imidazoline. Generally speaking any water soluble amphoteric or Zwitterionic surfactant compound which comprises a hydrophobic portion including a $C_{8-20}$ alkyl or alkenyl group and a hydrophilic portion containing an amine or quaternary ammonium group and a carboxylate, sulphate or sulphonic acid group may be used in our invention.

Compositions of our invention may also include cationic surfactants. The cationic surfactant may for example be an alkylammonium salt having a total of at least 8, usually 10 to 30, e.g. 12 to 24 aliphatic carbon atoms, especially a tri or tetra-alkylammonium salt. Typically alkylammonium surfactants for use according to our invention have one or at most two relatively long aliphatic chains per molecule (e.g. chains having an average of 8 to 20 carbon atoms each, usually 12 to 18 carbon atoms) and two or three relatively short chain alkyl groups having 1 to 4 carbon atoms each, e.g. methyl or ethyl groups, preferably methyl groups.

Typical examples include dodecyl trimethyl ammonium salts. Benzalkonium salts having one 8 to 20 C alkyl group two 1 to 4 carbon alkyl groups and a benzyl group are also useful.

Another class of cationic surfactants useful according to our invention are N-alkyl pyridinium salts wherein the alkyl group has an average of from 8 to 22, preferably 10 to 20 carbon atoms. Other similarly alkylated heterocyclic salts, such as N-alkyl isoquinolinium salts, may also be used.

Alkylaryl dialkylammonium salts, having an average of from 10 to 30 aliphatic carbon atoms are useful, e.g. those in which the alkylaryl group is an alkyl benzene group having an average of from 8 to 22, preferably 10 to 20 aliphatic carbon atoms and the other two alkyl groups usually have from 1 to 4 carbon atoms, e.g. methyl groups.

Other classes of cationic surfactant which are of use in our invention include alkyl imidazoline or quaternised imidazoline salts having at least one alkyl group in the molecule with an average of from 8 to 22 preferably 10 to 20 carbon atoms. Typical examples include alkyl methyl hydroxyethyl imidazolinium salts, alkyl benzyl hydroxyethyl imidazolinium salts, and 2 alkyl-1-alkylamidoethyl imidazoline salts.

Another class of cationic surfactant for use according to our invention comprises the amido amines such as those formed by reacting a fatty acid having 8 to 22 carbon atoms or an ester, glyceride or similar amide forming derivative thereof, with a di or poly amine, such as, for example, ethylene diamine or diethylene triamine, in such a proportion as to leave at least one free amine group. Quaternised amido amines may similarly be employed.

Alkyl phosphonium and hydroxyalkyl phosphonium salts having one $C_{3-20}$ alkyl group and three hydroxymethyl or methyl also be used as cationic surfactants in our invention.

Typically the cationic surfactant may be any water soluble compound having a positively ionised group, usually comprising a nitrogen atom, and either one or two alkyl groups each having an average of from 8 to 22 carbon atoms.

The anionic portion of the cationic surfactant may be any anion which confers water solubility, such as formate, acetate, lactate, tartrate, citrate, chloride, nitrate, sulphate or an alkylsulphate ion having up to 4 carbon atoms such as a methosulphate. It is preferably not a surface active anion such as a higher alkyl sulphate or organic sulphonate.

Polyfluorinated anionic, nonionic or cationic surfactants may also be useful in the compositions of our invention. Examples of such surfactants are polyfluorinated alkyl sulphates and polyfluorinated quaternary ammonium compounds.

Compositions of our invention may contain a semi-polar surfactant, such as an amine oxide, e.g. an amine oxide containing one or two (preferably one) $C_{3-22}$ alkyl group, the remaining substituent or substituents being preferably lower alkyl groups, e.g. $C_{1-4}$ alkyl groups or benzyl groups.

Particularly preferred for use according to our invention are surfactants which are effective as wetting agents, typically such surfactants are effective at lowering the surface tension between water and a hydrophobic solid surface. We prefer surfactants which do not stabilise foams to a substantial extent.

Mixtures of two or more of the foregoing surfactants may be used. In particular mixtures of non-ionic surfactants with cationic and/or amphoteric and/or semi polar surfactants or with anionic surfactants may be used. Typically we avoid mixtures of anionic and cationic surfactants, which are often less mutually compatible.

Preferably the biocide compound and the surfactant are present in a relative weight concentration of from 1:1000 to 1000:1, more usually 1:50 to 200:1, typically 1:20 to 100:1, most preferably 1:10 to 50:1, e.g. 1:1 to 20:1 especially 2:1 to 15:1.

Effective doses of the mixture are typically from 2 ppm to 2000 ppm more usually 20 ppm to 1,000 ppm e.g. 50 ppm to 500 ppm especially 100 to 250 ppm.

Compositions for use in water treatment may additionally or alternatively contain other biocides, oxygen scavengers, dispersants, antifoams, solvents, scale inhibitors, corrosion inhibitors and/or flocculants. In particular we have found a marked synergism between the posphonium biocides and thiocyano biocides such as MBT and TCMTB. Such mixtures are especially valuable as slimicides in paper making, eg for treatment of thin stock or back water.

Compositions according to our invention for use in controlling bryophites, lichens or fungal or microbial plant pathogens, contain an effective amount of a biocide as aforesaid, together with a horticulturally or agriculturally acceptable diluent, carrier and/or solvent therefor.

The phosphine compounds may be present as a solution in water at effective concentrations up to saturation. They will usually be supplied as concentrates at about 50 to 80% by weight concentration, e.g. 75% by wt. but will normally be diluted to a concentration of from 0.01 to 10% by wt. before application. Where damage to higher plants is to be avoided it is preferred to use concentrations below 1% w/w biocide, preferably below 0.2%. Alternatively the biocides may be admixed with or absorbed upon inert, particulate, non-phytotoxic solids such as talc or dissolved in organic solvents or suspended in or as dispersions or emulsions. They may be used in conjuntion with other moss killers or biocides, such as herbicides, fungicides, bactericides, insecticides and weedkillers, or with surfactants, wetting agents, adhesives, emulsifiers, suspending agents, thickeners, synergists, hormones, plant growth regulators or plant nutrients.

The compositions of our invention may be applied to lawns, flower or vegetable beds, arable land, meadowland, orchards or woodland, or hydroponic beds, or to the seeds, roots, leaves, flowers, fruit and/or stems of plants, or to paths, roads, walls, wood-work, brickwork or similar invasible surfaces.

The composition may be of value, inter alia, in controlling moss or sessile algae in lawns or on paths or walls, as seed dressings, as sprays for controlling fungal, bacterial or viral infections on leaves, flowers and fruit, such as mildew, botrytis, rust, fusarium, mosaic diseases or wilt, for application to soil or to the roots of seedlings (e.g. of brassica seedlings to inhibit club root) and in the control of numerous fungal, viral, protozoal and bacterial diseases of plants, including fungal blights such as potatoe blight, cankers such as apple canker, scabs, root rot, and base rot of bulbs. The compositions are especially effective in protecting cereal crops including wheat, barley, rye, oats, rice maize millet and sesame against a broad spectrum of plant diseases.

Other crops of importance which may be protected according to our invention include sugar cane; root vegetables including carrots, parsnips, turnips, beetroot, sugar beet, radishes, swedes and mangolds; brassicas including cabbages, broccoli, cauliflower and sprouts; grazing land; pulses including peas, broad beans, French, beans, runner beans, navy beans, kidney beans and lentils; curcubaceous plants including cucumber, marrows, gourds and squashes, oilseed rape, timber, rubber, cotton, coffee, cocoa, jute, tomatoes, potatoes, yams, tobacco, bananas, coconut palm, olives, alliums including onions, shalots, leeks, garlic, chives and spring onions, ground nuts, peanuts, sorghum, oil palm, roses, hemp, flax, lucerne, alfalfa, tea and fruit, including citrus fruit, apples, plums, peaches, nectarines, managoes, pears, cherries, grapes, berries, currants, dates, figs, avocados, almonds, and apricots.

The compounds are effective against a wide range of human and animal pathogens. They are especially useful in antiseptic/fungicidal creams and powders for treatment of Athletes Foot, Ringworm, Boils, and other infections and inflamations of the skin, in foot sprays, and in veterinary dips and udder washes.

The compounds may also be used in mouth washes and gargles for treating such condition as gum boils, Streptococcal infections and Oral Thrush. They are useful in pessaries and other preparations for treatment of Cystitis, Vaginal Thrush and other urinary and vaginal infections.

They may also be useful in topical, parenteral or oral preparations, for treatment, prevention and/or control of a variety of human and animal infections such as Salmonella infections in poultry and humans, Legionaires Disease, various types of Pneumonia, Staphylococcal infections, Rheumatic Fever, Plague, Scarlet Fever, Tuberculosis, Leprosy, Anthrax, protozoal infections, such as Amoebic Meningitis, Amoebic Dysentry, Malaria and Trypanosomiasis, and vermal infections such as Hookworm, Filariasis, Toxocariasis, Bilharzia,

Ascariasis, Tapeworm and Liver Fluke, and systemic fungal infections such as histoplasmosis, blastomycosis, paracoccidiomycosis, coccidiomycosis, and actinomycosis.

According to a further embodiment, therefore, our invention provides a pharmaceutical or veterinary composition comprising a pharmaceutically active proportion of a a biocide as foresaid and a pharmaceutically acceptable diluent, carrier or solvent therefore.

The composition may comprise an aqueous or organic solvent such as water, ethanol, isopropanol or glycerol, or mixtures thereof with a hydrocarbon, glyceride or other oil, preferably emulsified with water or an aqueous based solvent.

Particularly preferred are compositions adapted for topical application containing the biocide compound carried in a cream base or adsorbed on or absorbed in an inert particulate solid such as talc, kaolinite, bentonite, diatomaceous earth, zeolite or other absorbent aluminosilicate or calcium carbonate. Alternatively the compounds may be encapsulated or micro capsulated in a pharmaceutically acceptable encapsulant such as sugar, fat, gelatin, resin or gum, such as gum acacia, gum arabic, gum tragacanth or a slowly soluble synthetic polymer, such as polyvinyl alcohol or polyvinyl pyrrolidone or absorbed in a slow release glass such as a phosphate glass.

Alternatively the compounds or solutions thereof may respectively be dissolved in or emulsified with liquified gaseous solvents in pressurised containers. The invention also provides medicated shampoos and medicated soap in bar, liquid crystal or liquid form containing the biocide compounds.

The compounds may be tabletted with suitable solids such as chalk, kaolin, sugar or salt or dissolved in syrups, linctus or saline drips.

The compositions may additionally contain other fungicides, bactericides or synergists, antiperspirants, lanolin or other skin softening preparations, emulsifiers, dispersants, carboxymethyl cellulose, soaps, surfactants, polymeric thickening agents, wetting agents, foam controlling agents, perfumes, flavourings, colouring, deodorants, reodorants and antiseptics.

A typical ointment, for instance, may contain soft parafin, petroleum jelly, emulsifying wax, and/or wool fat, optionally in admixture with liquid parafin and/or hard parafin. A cream may typically comprise an aqueous based emulsion of emulsifying wax, together optionally with soft parafin or other hydrocarbons, and/or wool fat.

Creams may optionally contain buffers, such as citric acid/sodium phosphate. Ointments or creams may be combined with e.g. Zinc oxide or calamine to form pastes.

The effective dose depends upon the nature and site of the infection. Typically the biocide compounds exhibit bacterial and fungicidal activity at concentrations within the range 1 to 2000 ppm, especially 5 to 1000, preferably 10 to 500 e.g. 15 to 200 ppm. It is preferred to administer doses adapted to provide such concentrations at the site of infection, subject to toxicological constraints.

Compositions according to the invention typically contain from 0.2 to 20% by weight of the biocide compound preferably 0.5 to 10%. Aqueous solutions up to 80% by weight concentration are obtainable, but these normally require some dilution to provide compositions suitable to be administered to humans or animals. Our invention includes concentrates for dilution to provide pharmaceutical, veterinary or cosmetic preparations. Phosphonium salts are generally non-carcinogenic and non-mutagenic, but exhibit toxic effects especially on liver cells. Their toxicity in topical applications is generally low, but care must be taken in administering them internally.

The invention is of particular value for the treatment of vertebrates including: mammals such as cats, dogs and other carnivores, horses, sheep, pigs, cattle, goats, camels, deer and other ungulates, mice,hamsters, gerbils and other rodents, man and other primates; birds such as chickens, geese, ducks, turkeys and cage birds; reptiles; and fish.

The invention will be illustrated by the following examples:

## Example 1

THPC (9606g. 40.4 moles, commercial 80% solution) and water (3827g.) were charged to a reactor fitted with stirrer, a pressure equalising dropping funnel, gas inlet tube and condenser. The apparatus was flushed with nitrogen.

A solution of sodium hydroxide (1205g 30.1 moles) in water (2003g.) was added gradually from the dropping funnel to the well stirred THPC solution, the temperature being maintained below 40°C by cooling.

The mixture was heated to 48°C with vigorous stirring and methyl chloride introduced via the gas inlet tube. The methyl chloride was absorbed rapidly and temperature rose. After 2.5 hours methyl chloride absorption slowed down and the temperature had reached 68°C. A further portion of sodium hydroxide (10.3 moles. 414g.) in water (845g) was added. Methyl chloride addition was then continued for a further 3 hours until absorbance was zero.

The reactor was fitted for distillation and water-formaldehyde distilled off under vacuo at 50°C over 20 hours, extra water being added as required.

The concentrated solution of methyl tris hydroxy methyl phosphonium chloride was filtered to remove sodium chloride by-product to yield 7.417 Kg of 87.5% solution having the following n.m.r analysis:

| | |
|---|---|
| THPC | 7.3% |
| $(Me)_2\,P^+(CH_2OH)_2Cl^-$ | 0.3% |
| $CH_3\,P^+(CH_2OH)_3Cl^-$ | 92.9% |

## Example 2

Tris (acetoxymethyl) phosphine was prepared, according to the method of Mironova et al. Zhur. Obschei. Khim. 37 2747 (1967), by heating THP chloride with acetic anhydride in the presence of a catalytic amount of concentrated sulphuric acid at 90°C for 1.5 hours and evaporating acetic acid under vacuum, to form tetrakis (acetoxymethyl) phosphonium acetate which was then mixed slowly with aqueous sodium hydroxide in the presence of benzene, while cooling to maintain the temperature at 10-15°C. The reaction was performed under nitrogen.

The tris(acetoxymethyl) phosphine was dissolved in toluene and reacted with one equivalent of allyl bromide.

The allyl tris(acetoxymethyl) phosphonium bromide so formed was stirred for 2 hours at 20°C with 1 N hydrochloric acid to form allyl tris(hydroxymethyl) phosphonium bromide in high yield and purity. The product was characterised by $^{31}$P.n.m.r.

Example 3

Dry THPC (190.5g 1 mole) was dissolved in methanol (300mls) and sodium methoxide solution (0.98 mole NaOMe) added. The solution was stirred for one hour at ambient temperature and allyl bromide (121g. 1 mole) added. After allowing to react at ambient temperature overnight the solution was filtered to remove sodium chloride and the filtrate stripped under vacuum to remove methanol and formaldehyde. The product was a viscous oil of $^{31}$P.N.m.r. analysis:

| | |
|---|---|
| THPC | 5% |
| Allyl tris (hydroxymethyl) phosphonium bromide | 75% |
| diallyl bis (hydroxymethyl) phosphonium bromide | 20% |

Example 4

Tris (hydroxymethyl) phosphine in methanol was treated with one equivalent of ethyl iodide and the resulting mixture heated at reflux for 6 hours. The resulting mixture of phosphonium compounds, $(CH_2OH)_4PI$, $EtP(CH_2OH)_3I$ and $Et_2$ $P(CH_2OH)_2$ I was acetylated by treatment with acetic anhydride using sulphuric acid catalyst. Conversion of the acetylated phosphonium compounds to a mixture of acetylated phosphines, $(AcOCH_2)_3$ P, $EtP(CH_2OAc)_2$ and $Et_2$ P $CH_2OAc$, was as done in example 2.

Fractional distillation gave pure ethyl di(acetoxymethyl) phosphine which was treated with formaldehyde and excess IN HCl to yield ethyl tris(hydroxymethyl) phosphonium Et $P(CH_2OH)_3$ chloride which was characterized by $^{31}$P.N.m.r.

Example 5

Ethyl tris (hydroxymethyl) phosphonium chloride (Ethyl THPC) prepared according to the method of example 4, THPS, and a tetrakis (1 - hydroxy ethyl) phosphonium chloride (comparative example) were each dosed at four different concentrations to separate samples of an infected cooling water.

Bacterial counts per ml. of solution were estimated after 17 hours. The results are set out in Table 1

Table 1

| | 50 ppm | 100 ppm | 150 ppm | 200 ppm |
|---|---|---|---|---|
| CONTROL (NO BIOCIDE) | ($10^6/10^6$ DUPLICATE MEASUREMENTS) | | | |
| ETHYL THPC | $10^3$ | 0 | 0 | 0 |
| THPS (COMPARATIVE) | $10^5$ | $10^4$ | 0 | 0 |
| THEPC (COMPARATIVE) | $10^6$ | $10^6$ | $10^6$ | $10^6$ |

The results show that the comparative example exhibited no bactericidal activity in this test, THPS was effective but the Ethyl THPC was more effective than THPS at lower concentrations.

Example 6

Methyl tris (hydroxy methyl) phosphonium chloride (methyl THPC), THPS, and two other comparative examples, cyclohexyl tris (hydroxy methyl) phosphonium chloride and octyl tris (hydroxy methyl) phosphonium sulphate were each added at concentrations of 50, 200 and 1,000 ppm to separate samples of an infected cooling water. The control samples without added biocide each showed a bacterial count per ml. of $10^7$ after 17 hours; the remaining samples gave bacterial counts per ml. as shown in Table 2

Table 2

| | 50 ppm | 200 ppm | 1000 ppm |
|---|---|---|---|
| CYCLOHEXYL THPC (COMPARATIVE) | $10^7$ | $10^7$ | $10^6$ |
| OCTYL THPS (COMPARATIVE) | $10^7$ | $10^7$ | $10^6$ |
| THPS (COMPARATIVE) | $10^7$ | 0 | 0 |
| METHYL THPC | $10^3$ | 0 | 0 |

The experiment confirms that longer chain or cyclic aliphatic phosphonium salts are ineffective, but that methyl tris (hydroxy methyl) phosphonium salts are even more active at low concentrations than THPS.

Example 7

Allyl tris (hydroxymethyl) phosphonium bromide (allyl THPB) prepared as described in Ex3 was compared with 2, 6-dichlorobenzyl tris (hydroxymethyl) phosphonium bromide and THPS in a biocide test as described in example 5. The results are shown in Table 3

Table 3

| | 50 ppm | 100 ppm | 150 ppm | 200 ppm |
|---|---|---|---|---|
| 2, 6-DICHLOROBENZYL (THPB) | $10^6$ | $10^6$ | $10^6$ | $10^6$ |
| THPS | $10^3$ | 0 | 0 | 0 |
| ALLYL THPB | $10^3$ | 0 | 0 | 0 |

The experiment confirms that aromatic phosphonium salts are ineffective as bactericides, but that allyl THPB performs at least as well as THPS.

Example 8

$(HOCH_2)_3 P^+CH_2CH_2P^+(CH_2OH)_3 Cl_2^-$

A solution of ethylene bis phosphonous dichloride ($Cl_2PCH_2CH_2PCl_2$, 10g.) in dry diethyl ether (50 mls.) was added over a period of one hour to a stirred suspension of $LiAlH_4$ (4.0g) in diethyl ether (100mls.) cooled at 10°C. The reaction mixture was stirred overnight at ambient temperature and the excess of $LiAlH_4$ destroyed by adding isopropanol and water. The ether solution was separated and shown by $^{31}P$ nmr to contain the desired ethylene diphosphine, $H_2PCH_2CH_2PH_2$. The ether solution was reacted at 20 - 40°C overnight with a slight excess of aqueous formaldehyde and aqueous hydrochloric acid. The aqueous solution was stripped under vacuo to give the desired phosphonium compound as a colourless solid m.p.173-4°C. Found P 17.4%, Calc P 17.8%. The product was characterised by $^{31}P$ and $^{13}C$ nmr.

Example 9

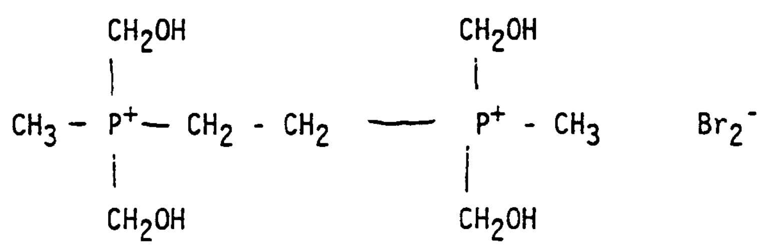

Methyl bis acetoxy methyl phosphine ($CH_3P(CH_2OAc)_2$, 28g.) was added to ethylene dibromide (13.2g) in 100 mls of deoxygenated dimethyl formamide and the resulting solution was refluxed under nitrogen for 10 hours at 120-125°C. The product, a white crystalline solid, precipitated as the reaction progressed. Filtration gave the desired intermediate ethylene bis(methyl bis acetoxymethyl phosphonium bromide 36g. 88% yield. The acetoxy compound was dissolved in 1N hydrochloric acid (300ml) and stirred overnight at ambient temperature to effect hydrolysis of the acetate groups before stripping under vacuo to give desired product, a white crystalline solid m.p. 165-166°C (21.3g, 75% yield overall). The product was characterised by $^{31}P$ and $^{13}C$ n.m.r. and elemental analysis.
Found: P, 14.9%; Br, 38.0%; C,21.4% Calc: P 15.4%, Br, 39.6%; C 23.8%.

Example 10

Ethylene bis (methyl bis hydroxymethyl phosphonium) bromide (EBMB) made by the process of Example 8, and ethylene bis (tris hydroxy methyl phosphonium) chloride (EBTC) made by the process of Example 8 were tested as biocides in the manner described in Example 5. The results are shown in Table 4.

Table 4

| | 50 ppm | 100 ppm | 150 ppm | 200 ppm |
|---|---|---|---|---|
| EBMB | $10^5$ | $10^4$ | $10^3$ | $10^3$ |
| EBTC | $10^4$ | $10^{3.5}$ | $10^3$ | $10^3$ |

**Claims**

1. The use as a biocide of a phosphonium salt of the formula:
$$[R\ P\ (CH_2OH)_3]^+\ 1/v\ X^{(v-)}$$
wherein R is a methyl, ethyl or allyl group and X is an anion having a valency v such that the salt is water soluble, or a water soluble condensate thereof with ammonia or an amine or amide.

2. The use as a biocide of a water soluble methyl tris (hydroxymethyl) phosphonium salt.

3. The use as a biocide of a water soluble ethyl tris (hydroxymethyl) phosphonium salt.

4. The use as a biocide of a water soluble allyl tris (hydroxymethyl) phosphonium salt.

5. A method of disinfecting or preventing infection of a locus infected with, or infectable by microorganisms that comprises applying to said locus a biocide as specified in any of Claims 2 to 4.

6. A method according to Claim 5 wherein the biocide is a chloride, sulphate, phosphate, sulphite, phosphite, bromide, nitrate, borate, acetate, formate, lactate, methosulphate, citrate or carbonate.

7. A method according to either of Claims 5 and 6 for treating cooling water, boiler water, process water, geothermal water, central heating systems or air conditioning systems.

8. A method according to either of Claims 5 and 6 for treating swimming pools, fish ponds, aquaria, fish farms, lakes, streams, canals, or reservoirs.

9. A method according to either of Claims 5 and 6 for treating oil field produced water, injection water, drilling fluids, or water for hydrostatic testing.

10. A method according to either of Claims 5 and 6 for disinfecting farmyards, homes, hospitals or surgeries.

11. A method according to either of Claims 5 and 6 for preserving aqueous based formulations.

12. A method according to either of Claims 5 and 6 for fumigation of grain silos, stored crops or crop storage areas.

13. A method according to either of Claims 5 and 6 for killing moss.

14. A method according to either of Claims 5 and 6 for protecting growing plants from plant pathogens.

15. Use of a biocide as specified in any of Claims 2 to 4 to prepared pharmaceutical or veterinary compositions.

## Patentansprüche

1. Die Verwendung als Biozid eines Phosphoniumsalzes der Formel:
$$[R\,P\,(CH_2OH)_3]^+\ 1/v\ X^{(v-)}$$
in der R eine Methyl-, Ethyl- oder Allylgruppe und X ein Anion mit der Valenz v ist, so daß das Salz wasserlöslich ist, oder eines wasserlöslichen Kondensats desselben mit Ammoniak oder einem Amin oder Amid.

2. Die Verwendung als Biozid eines wasserlöslichen Methyl-tris-(hydroxymethyl)-phosphoniumsalzes.

3. Die Verwendung als Biozid eines wasserlöslichen Ethyl-tris-(hydroxymethyl)-phosphoniumsalzes.

4. Die Verwendung als Biozid eines wasserlöslichen Allyl-tris-(hydroxymethyl)-phosphoniumsalzes.

5. Verfahren zur Desinfektion oder zur Vermeidung einer Infektion eines Ortes, der mit Mikroorganismen infiziert ist oder infiziert werden kann, welches darin besteht, daß man auf diesen Ort ein Biozid gemäß einem der Ansprüche 2 bis 4 aufbringt.

6. Verfahren nach Anspruch 5, bei dem das Biozid ein Chlorid, Sulfat, Phosphat, Sulfit, Phosphit, Bromid, Nitrat, Borat, Acetat, Formiat, Lactat, Methosulfat, Citrat oder Carbonat ist.

7. Verfahren nach einem der Ansprüche 5 und 6 zur Behandlung von Kühlwasser, Boilerwasser, Betriebswasser, geothermischem Wasser, Zentralheizungs- oder Klimaanlagen.

8. Verfahren nach einem der Ansprüche 5 und 6 zur Behandlung von Schwimmbecken, Fischteichen, Aquarien, Fischfarmen, Seen, Flüssen, Kanälen oder Wasserreservoiren.

9. Verfahren nach einem der Ansprüche 5 und 6 zur Behandlung von auf Ölfeldern anfallendem Wasser, Flutwasser, Spülschlämmen oder Wasser für hydrostatische Tests.

10. Verfahren nach einem der Ansprüche 5 und 6 zur Desinfektion von Wirtschaftshöfen, Haushalten, Krankenhäusern oder Operationssälen.

11. Verfahren nach einem der Ansprüche 5 und 6 zur Konservierung von Formulierungen auf wässeriger Basis.

12. Verfahren nach einem der Ansprüche 5 und 6 zur Begasung von Getreidesilos, gespeicherten Ernteerträgen oder von Bereichen, in denen die Ernte gespeichert wird.

13. Verfahren nach einem der Ansprüche 5 und 6 zur Abtötung von Moosen.

14. Verfahren nach einem der Ansprüche 5 und 6 zum Schutz wachsender Pflanzen vor Pflanzenpathogenen.

15. Die Verwendung eines Biozids gemäß einem der Ansprüche 2 bis 4 zur Herstellung pharmazeutischer oder veterinärmedizinischer Zusammensetzungen.

## Revendications

1. Utilisation comme biocide d'un sel de phosphonium de formule:
$$[R\,P\,(CH_2OH)_3]^+\ 1/v\ X^{(v-)}$$
dans laquelle R est un radical méthyle, éthyle ou allyle et X est un anion ayant une valence v telle que le sel est hydrosoluble ou un condensé hydrosoluble de celui-ci avec de l'ammoniac, une amine ou un amide.

2. Utilisation comme biocide d'un sel hydrosoluble de méthyl-tris(hydroxyméthyl)phosphonium hydrosoluble.

3. Utilisation comme biocide d'un sel hydrosoluble d'éthyl-tris(hydroxyméthyl)phosphonium.

4. Utilisation comme biocide d'un sel hydrosoluble d'allyl-tris(hydroxyméthyl)phosphonium.

5. Méthode de désinfection ou de prévention d'infections d'un site infecté ou susceptible de l'être par des microorganismes qui comprend l'application sur ledit site d'un biocide spécifié dans l'une des revendications 2 à 4.

6. Méthode selon la revendication 5 dans laquelle le biocide est un chlorure, un sulfate, un phosphate, un sulfite, un phosphite, un bromure, un nitrate, un borate, un acétate, un formiate, un lactate, un méthosulfate, un citrate ou un carbonate.

7. Méthode selon une des revendications 5 à 6 pour le traitement de l'eau de refroidissement, de l'eau de chaudières, de l'eau de processus industriels, de l'eau géothermique, de systèmes de chauffage central ou de conditionnement d'air.

8. Méthode selon une des revendications 5 à 6 pour le traitement des piscines, des viviers, des aquariums, des centres de pisciculture, des lacs, des cours d'eau, des canaux ou des réservoirs.

9. Méthode selon une des revendications 5 à 6 pour le traitement de l'eau produite dans des champs pétrolifères, de l'eau d'injection, des fluides de forage ou de l'eau destinée aux tests hydrostatiques.

10. Méthode selon une des revendications 5 à 6 pour la désinfection des cours de ferme, des maisons, des hôpitaux ou des services de chirurgie.

11. Méthode selon une des revendications 5 à 6 pour la préservation de formules aqueuses.

12. Méthode selon une des revendications 5 à 6 pour la fumigation de silos de céréales, des récoltes stockées ou des entrepôts de stockage des récoltes.

13. Méthode selon une des revendications 5 à 6 pour tuer la mousse.

14. Méthode selon une des revendications 5 à 6 pour la protection des végétaux en cours de croissance contre des agents pathogènes des végétaux.

15. Utilisation d'un biocide selon une des revendications 2 à 4 dans des préparations pharmaceutiques ou vétérinaires.